(19) Europäisches Patentamt / European Patent Office / Office européen des brevets

(11) **EP 1 749 190 B1**

(12) **EUROPÄISCHE PATENTSCHRIFT**

(45) Veröffentlichungstag und Bekanntmachung des Hinweises auf die Patenterteilung:
**06.05.2015 Patentblatt 2015/19**

(51) Int Cl.:
**G01B 21/04** *(2006.01)*     **G01B 15/04** *(2006.01)*
**G01N 23/083** *(2006.01)*     **A61B 6/00** *(2006.01)*

(21) Anmeldenummer: **05750966.3**

(22) Anmeldetag: **24.05.2005**

(86) Internationale Anmeldenummer:
**PCT/EP2005/005598**

(87) Internationale Veröffentlichungsnummer:
**WO 2005/119174 (15.12.2005 Gazette 2005/50)**

(54) **Verfahren zum Messen eines Objekts mit einem Koordinatenmessgerät, das einen Computer-Tomographen enthält**

Method for measuring an object using a coordinate measuring machine including a computer tomograph

Procédé de mesure d'un objet utilisant un appareil de mesure de coordonnées incluant un tomodensitomètre

(84) Benannte Vertragsstaaten:
**AT BE BG CH CY CZ DE DK EE ES FI FR GB GR HU IE IS IT LI LT LU MC NL PL PT RO SE SI SK TR**

(30) Priorität: **26.05.2004 DE 102004026357**
**14.10.2004 DE 102004050257**
**20.04.2005 DE 102005018447**

(43) Veröffentlichungstag der Anmeldung:
**07.02.2007 Patentblatt 2007/06**

(60) Teilanmeldung:
**10002412.4 / 2 192 380**
**10184423.1 / 2 282 165**

(73) Patentinhaber: **Werth Messtechnik GmbH**
**35394 Giessen (DE)**

(72) Erfinder:
• **CHRISTOPH, Ralf**
**35394 Giessen (DE)**
• **RAUH, Wolfgang**
**35094 Lahntal-Großfelden (DE)**

(74) Vertreter: **Stoffregen, Hans-Herbert**
**Patentanwalt**
**Friedrich-Ebert-Anlage 11b**
**63450 Hanau (DE)**

(56) Entgegenhaltungen:
EP-A- 0 343 600     EP-A- 1 313 065
EP-A- 1 380 263     EP-A- 1 380 263
FR-A- 2 721 497     FR-A- 2 721 497
US-A1- 2005 151 963

• BAUER WALTER ET AL: "Computer tomography for non-destructive testing in the automotive industry" PROC SPIE INT SOC OPT ENG; PROCEEDINGS OF SPIE - THE INTERNATIONAL SOCIETY FOR OPTICAL ENGINEERING; DEVELOPMENTS IN X-RAY TOMOGRAPHY IV 2004, Bd. 5535, 2004, Seiten 464-472, XP002340338
• BAUER WALTER ET AL: "Computer tomography for non-destructive testing in the automotive industry" PROC SPIE INT SOC OPT ENG; PROCEEDINGS OF SPIE - THE INTERNATIONAL SOCIETY FOR OPTICAL ENGINEERING; DEVELOPMENTS IN X-RAY TOMOGRAPHY IV 2004, Bd. 5535, 2004, Seiten 464-472, XP002340338
• PATENT ABSTRACTS OF JAPAN Bd. 2002, Nr. 06, 4. Juni 2002 (2002-06-04) & JP 2002 055062 A (SHIMADZU CORP), 20. Februar 2002 (2002-02-20)
• PATENT ABSTRACTS OF JAPAN Bd. 018, Nr. 638 (P-1837), 5. Dezember 1994 (1994-12-05) -& JP 06 249641 A (TOKYO SEIMITSU CO LTD), 9. September 1994 (1994-09-09)

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 1 749 190 B1

**Beschreibung**

Die Erfindung bezieht sich auf ein Verfahren zum Messen eines Objekts mit einem Koordinatenmessgerät enthaltend zumindest einen Computer-Tomographen mit Röntgenquelle und Röntgendetektor.

Zum Messen der Geometrie von Werkstücken sind Koordinatenmessgeräte mit verschiedenen Sensoren bekannt. Als solche Sensoren werden optische und taktile Sensoren beschrieben (DE.Z.: Die Bibliothek der Technik, Bd. 248). Ebenfalls ist der Einsatz von Computer-Tomographen zur Bestimmung von Werkstückgeometrien insbesondere von Fehlstellen bekannt. So wird in der DE-A- 103 31 419 eine Kombination beider Geräte beschrieben. Hierbei ist ein Computer-Tomograph fest am Grundaufbau des Koordinatenmessgerätes befestigt. Dabei wird mittels klassischer Koordinatenmesstechnik-Sensorik die Position des Messobjektes bestimmt und hierauf folgend in den Messbereich des Computer-Tomographen positioniert.

Beim beschriebenen Stand der Technik finden verschiedene Aufgabenstellungen keine Beachtung. Ungelöst bleibt zum Beispiel das Problem, dass die Messobjekte eine größere Ausdehnung aufweisen können als der Messbereich des Computer-Tomographen. Da dieser starr am Grundaufbau des Koordinatenmessgerätes befestigt ist, ist ein Zusammensetzen mehrerer Computertomographie-Aufnahmen nicht möglich.

Darüber hinaus weisen Computer-Tomographen üblicherweise eine relativ grobe Messunsicherheit in der Größenordnung von 10 $\mu$m oder mehr auf. Die alleinige Messung des Messobjektes mit dem Computer-Tomographen - wie in der DE-A- 103 31 419 beschrieben - ist somit zur vollständigen Lösung der geometrischen Messaufgaben an üblichen Zeichnungsteilen nicht ausreichend. Ein weiteres Problem besteht darin, die geometrische Kalibrierung der Computer-Tomographen vorzunehmen. Da die Eigenschaften der tomographischen Messung stark von den Eigenschaften des Messobjektes selbst abhängen, ist dies nur schwer global an Messnormalen durchzuführen.

Aus der DE-A-100 44 169 ist ein Verfahren zur Bestimmung der Dicke von Werkstücken bekannt. Dabei trifft die ein zu messendes Bauteil durchsetzende Röntgenstrahlung auf einen Detektor. Das Bauteil kann mittels eines Manipulators gedreht sowie angehoben und abgesenkt werden. Nach vollständiger Durchleuchtung des Bauteils liefert ein Rechner eines Computer-Tomographen einen Stapel von Grauwertschnittbildern, die zusammengefügt werden, um einen dreidimensionalen Voxel-Datensatz zu erhalten. Aus diesem wird sodann die Wandstärke des Bauteils berechnet.

Aus der DE-C-38 06 686 ist ein Koordinatenmessgerät mit einem Multisensortastsystem bekannt, das einen taktil arbeitenden Sensor, einen Lasersensor und einen Videosensor umfasst, wobei entsprechend den Messaufgaben ein Einsatz einer der Sensoren erfolgt. Einen dieser Sensoren durch einen Computer-Tomographen zu ersetzen, sieht die EP-A-1 380 263 vor.

Bei der Verwendung einer Röntgensensorik sind umfangreiche Schutzmaßnahmen zur Abschinnung der Röntgenstrahlen erforderlich, um Strahlenschutzvorschriften zu genügen. Dabei muss sichergestellt sein, dass die Strahlenbelastung außerhalb der Messanordnung vorgegebene Grenzwerte nicht überschreitet. Um diesen Anforderungen zu genügen, ist es bekannt, dass um die Messanordnung, also unabhängig von dieser ein Strahlenschutzgehäuse angeordnet wird, das z. B. aus Blei oder Bleischichten aufweisendem Verbundmaterial besteht. Das Strahlenschutzgehäuse hat dabei die ausschließliche Aufgabe, die im Computer-Tomographen entstehende Röntgenstrahlung zu absorbieren. Durch das zusätzliche Gehäuse wird die Gesamtmessanordnung voluminös. Eine unerwünschte Gewichtserhöhung sowie hohe Kosten sind weitere Folgen.

Nachteil bekannter Computer-Tomographen ist es des Weiteren, dass die Messgeschwindigkeit hinter der zurückbleibt, die in der Koordinatenmesstechnik mit optischen Sensoren erzielbar ist. Nachteilig ist es auch, dass der Computer-Tomograph fest am Grundaufbau des Koordinatenmessgerätes befestigt sind, so dass die Mess-Einsatzmöglichkeit eingeschränkt ist.

Aus der US-A-2003/0043964 ist ein Inspektionssystem für Flugzeugrümpfe bekannt, das eine innerhalb des Rumpfes von einem Kran ausgehende Röntgenquelle und einen außerhalb des Rumpfes ebenfalls von einem Kran ausgehenden Sensor umfasst. Um die Position des Sensors zu bestimmen, wird ein Triangulatiönsverfahren benutzt.

Ein Messgerät nach der DE-A-100 01 239 sieht neben einem Positionsdetektor ein nicht optisches Messsystem wie ein AFM (Atomic Force Microscope) vor, die über ein Tragelement starr miteinander verbunden sind.

Ein Multisensormesskopf nach der DE-A-44 45 331 umfasst eine Vertikalachse, auf der mehrere Sensoren montiert sein können.

In einem Koordinatenmessgerät nach der EP-A-0 504 609 werden neben Messköpfen auch Gelenkfräsköpfe eingesetzt.

Eine Röntgenprüfanordnung nach der US-A-5,038,378 sieht die Möglichkeit vor, einen Röntgenstrahldetektor unabhängig voneinander entlang von drei Achsen zu verstellen.

Der vorliegenden Erfindung liegt die Aufgabe zu Grunde, ein Verfahren und ein Koordinatenmessgerät zum Messen eines Objektes mit zumindest einer Röntgensensorik derart weiterzubilden, dass im Vergleich zum Stand der Technik eine höhere Messsicherheit erzielbar sein soll. Des Weiteren soll auf einfache Weise eine geometrische Kalibrierung des Computer-Tomographen ermöglicht werden.

Die Aufgabe wird durch das Verfahren des Anspruchs 1 gelöst. Bevorzugte Verfahren ergeben sich aus den Ansprüchen 2 - 31.

Um eine hohe Messsicherheit zu erzielen und auf einfache Weise eine geometrische Kalibrierung des Computer-Tomographen zu ermöglichen, schlägt die Erfindung vor, dass

Mit anderen Worten ist erfindungsgemäß vorgesehen, dass alle Einstellparameter, die für das Tomographieren mit einer bestimmten Vergrößerung oder einem bestimmten Messbereich notwendig sind - hierzu gehört die Position für Röntgenquelle und Röntgendetektor die Position der verschiedenen Achsen des Tomographie- bzw. Koordinatenmessgerätes -, sowie die diesen Positionen zugeordneten Vergrößerungswerte und andere Kalibrierdaten inklusive Korrekturwerte zur Position der Achsen bezüglich des durch die zugeordneten Wegmesssysteme gelieferten Positionsmesswertes in einem einmaligen Einmessvorgang des Koordinatenmessgerätes messtechnisch erfasst und abgespeichert werden. Die so gespeicherten Daten werden oder weitere Einmessvorgänge für nachfolgende Messungen mit dem Computer-Tomographen benutzt. Diese abgespeicherten Werte werden sodann bei normalem Betrieb des Koordinatenmessgerätes durch Knopfdruck des Bedieners bzw. durch Aufruf aus einem CNC-Programm abgerufen, die Maschinen in die entsprechenden Positionen verfahren und die zugehörigen Kalibrierdaten beim nachfolgenden Messprozess benutzt.

Insbesondere ist vorgesehen, dass vorab eingemessene Vergrößerungs- und Messbereichseinstellungen durch das Messprogramm des Koordinatenmessgerätes automatisch aufgerufen und die entsprechenden Hardware-Komponenten des Gerätes positioniert werden.

Ferner besteht die Möglichkeit, dass Röntgenquelle und Röntgendetektor synchron verfahren werden, um lediglich die Vergrößerung und/oder Messbereich zu verändern bzw. dass Röntgenquelle und Röntgendetektor unabhängig voneinander verfahren werden, um Vergrößerung und/oder Messbereich zu verändern.

Es besteht auch die Möglichkeit, dass alle für das Röntgenmessen (Tomographieren) notwendigen Einstellungen im Vorhinein eingemessen und abgespeichert werden und beim jeweiligen Röntgenmessvorgang wie Tomographiervorgang keinerlei Einmessvorgänge mehr notwendig sind.

Die Drehmittelpunktjustage kann durch einen Einmessvorgang und/oder eine entsprechende Korrektur des Drehmittelpunktversatzes in der Software realisiert werden.

Eine Weiterbildung sieht vor, dass die Bestimmung der Vergrößerung für die Tomographie und/oder der Drehmittelpunktlage in Bezug auf Röntgenquelle und Röntgendetektor mittels eines Normals bestimmt wird, das aus mindestens zwei Kugeln besteht. Insbesondere ist vorgesehen, dass das Normal aus vier Kugeln besteht.

Ein Verfahren zur Bestimmung der Position des Drehmittelpunkts im Koordinatenmessgerät zeichnet sich insbesondere durch die Verfahrensschritte aus

- ein Vierkugelnormal bestehend aus vier in den Ecken eines Rechtecks wie Quadrats angeordneten Kugeln, bei dem die Abstände der Kugeln zueinander bekannt bzw. kalibriert sind, wird auf der Drehachse positioniert,
- das Vierkugelnormal wird so verdreht, dass die aufgespannte Ebene parallel zum Detektor steht,
- Messung der Vierkugelposition im Messfeld des Detektors,
- Berechnung der mittleren Vergrößerung M1 aus den vier gemessenen Kugeldistanzen, den nominalen Kugeldistanzen und der nominalen Pixelgröße des Detektors,
- Drehung der Drehachse um 180°,
- Messung der vier Kugelpositionen im Bild,
- Berechnung der mittleren Vergrößerung M2 aus den vier gemessenen Kugeldistanzen, den nominalen Kugeldistanzen und der nominalen Pixelgröße des Detektors.

Die Berechnung der Y-Position des Drehmittelpunktes anhand der vier Kugelpositionen vor und nach der Drehung erfolgt nach folgender Gleichung: Pdyn = (Pkyn1 * M2 + Pkyn2 * M1) / (M1 + M2) mit Pdyn: Y-Position der Drehachse auf dem Detektor für Kugel n, Pkyl : Y-Position der Kugel n ei Drehwinkel 0°, Pkyn2: Y-Position der Kugel n bei Drehwinkel 180°, M1: mittlere Vergrößerung bei Drehwinkel 0°, M2: mittlere Vergrößerung bei Drehwinkel 180°.

Es besteht auch die Möglichkeit, dass das Messen eines Objektes mit einem Koordinatenmessgerät erfolgt, das neben der Röntgensensorik (Computer-Tomographen) weitere Sensoren aufweist, so dass Messungen mittels einer taktilen und/oder optischen Sensorik durchgeführt werden können, wobei insbesondere taktil-optische Messungen zu erwähnen sind. Somit besteht die Möglichkeit, dass eine Korrektur der mit der Röntgensensorik bzw. Tomographie gemessenen Messpunktewolke des Messobjektes oder der hieraus errechneten triangulierten Flächenelemente durch taktil oder optisch gewonnene Messpunkte erfolgt, wobei zwischen den taktil und/oder optisch gemessenen Korrekturpunkten interpoliert werden kann.

Auch kann zwischen den mit taktiler und/oder optischer Sensorik gewonnenen Korrekturpunkten unter Berücksichtigung des Funktionsverlaufs der durch Röntgenmessung wie Tomographie gewonnenen Punktewolke interpoliert werden und/oder durch Berücksichtigung des Nominal-CAD-Models interpoliert werden.

Ein weiterer Vorschlag sieht vor, dass zuerst ein Musterteil des Messobjekttyps mittels Röntgenstrahlen (tomographisch) und taktil und/oder optisch abgetastet wird, aus der Differenz beider Messungen ein Korrekturnetzwerk für die Korrektur der tomographischen Messwerte errechnet wird und beim Messen von Serienteilen die tomographischen Messungen mit den einmalig bestimmten Korrekturwerten korrigiert werden.

Die diesbezügliche Vorgehensweise ist so zu verstehen, dass in einem ersten Messvorgang viele Messpunkte zu einem typischen Vertreter des Messobjektes mittels Röntgenstrahlen tomographisch und taktil oder optisch gemessen werden. Hierbei wird auch beim taktilen und/oder optischen Messen mit sehr vielen Messpunkten gearbeitet, um ein ausreichend dichtes Korrekturnetzwerk zu erzielen. Hieraus werden sodann für jeden Oberflächenort des zu tomographierenden Objektes entsprechende Korrekturwerte ermittelt, die sich aus dem Vergleich der taktilen und/oder optischen Messwerte mit den tomographischen Messwerten ergeben. Beim späteren Tomographieren von weiteren Teilen werden diese Korrekturwerte direkt zum Ansatz gebracht. Ein nochmaliges taktiles oder optisches Gegenmessen ist nicht erforderlich.

Folglich ist vorgesehen, dass ein kalibriertes Teil eines Messobjektes tomographiert wird und aus der Messabweichung bei der Messung ein Korrekturnetzwerk für die Korrektur der tomographischen Messwerte errechnet wird und beim Messen von Serienteilen die tomographischen Messungen mit den vorher abgestimmten Korrekturwerten abgestimmt werden.

Bei der Messung von Serienteilen können zusätzlich einzelne optisch und/oder taktil gemessene Korrekturpunkte berücksichtigt werden.

Die taktilen und/oder optischen Messpunkte können zur Korrektur durch einen Bediener grafisch an der durch Tomographie bestimmten Punktwolke festgelegt werden und durch das Koordinatenmessgerät dann automatisch gemessen werden.

Eine Weiterbildung sieht vor, dass die taktilen und/oder optischen Messpunkte zur Korrektur durch einen Bediener grafisch an dem dem zu messenden Teil zugrunde liegenden CAD-Modell festgelegt werden und durch das Koordinatenmessgerät dann automatisch gemessen werden. Dabei können die taktilen und/oder optischen Messpunkte zur Korrektur durch einen automatischen Algorithmus auf der Oberfläche des dem zu messenden Teil zugrunde liegenden CAD-Modells annähernd gleichmäßig oder gleichmäßig verteilt durch das Koordinatenmessgerät automatisch gemessen werden. Auch können die taktilen und/oder optischen Messpunkte zur Korrektur durch einen Bediener grafisch am CAD-Modell festgelegt und nach dem Laden des CAD-Modells durch das Koordinatenmessgerät automatisch gemessen werden.

Die Erfindung lehrt des Weiteren, dass beim Tomogräphiervorgang grundsätzlich ein Kalibrierkörper, insbesondere eine Anordnung von Kugeln mit tomographiert wird und hieraus die Relativlage der Drehachse zum Koordinatenmessgerät und/oder zur Röntgenquelle und/oder zum Röntgensensor bestimmt und anschließend mathematisch korrigiert wird. Dabei kann die Lage der Kalibrierkörper auf der Drehachse mit optischen und/oder taktilen Sensoren bestimmt und zur Korrektur der Lage der Drehachse herangezogen werden.

Insbesondere ist jedoch vorgesehen, dass der Kalibrierkörper wie die zumindest zwei Kalibrierkugeln in der Halterung, d. h. dem Drehtisch des zu messenden Objektes in einem Material eingelagert sind, das eine geringe Absorption gegenüber Röntgenstrahlung aufweist. Somit kann durch die Erfassung des Kalibrierkörpers das Messobjekt auf dem Drehtisch positioniert werden, da mittels des Kalibrierkörpers die Drehpunktlage, also die Drehachse des Drehtisches ermittelt werden kann.

Die Raumlage der Rotationsachse in Bezug zur Röntgenquelle und Röntgendetektor kann erfindungsgemäß mit der Röntgensensorik und/oder mit der taktilen Sensorik und/oder mit der optischen Sensorik messtechnisch bestimmt und diese Lageabweichung beim Tomographieren von Messobjekten mathematisch korrigiert werden.

Insbesondere wird die von der Nominallage abweichende Lage der Drehachse durch Rotation und/oder Translation und/oder Verzerrung der 2D-Einzelbilder korrigiert.

Des Weiteren kann die von der Nominallage abweichende Lage der Drehachse im Rekonstruktionsalgorithmus berücksichtigt werden.

Ein weiterer Vorschlag der Erfindung sieht vor, dass das Messobjekt durch Einsatz von taktilen und/oder optischen Sensoren und/oder Tomographie in seiner Lage auf dem Drehtisch des Messgerätes und somit im Maschinenkoordinatensystem bestimmt wird und anschließend im 2D-Durchstrahlungsmodus an eingemessener Position des Röntgensensors durch Maße mit Methoden der Bildverarbeitung gemessen werden.

Durch diese Maßnahmen wird ein weiteres Nutzungsprinzip des erfindungsgemäßen Koordinatenmessgerätes, in dem eine Röntgensensorik integriert ist, verdeutlicht. So wird die Möglichkeit geschaffen, in 2D-Durchstrahlungsbildern zu messen. Üblicherweise ist dies nicht möglich, da die aktuelle Vergrößerung am Messobjekt nicht bekannt ist; denn die Position des Messobjekts im Röntgenstrahlengang ist unbekannt, die Position jedoch die Vergrößerung nach dem Strahlensatz wesentlich bestimmt. Wird nun die Position des Messobjekts im Koordinatenmessgerät mit einer optischen und/oder taktilen Sensorik genau bestimmt, ist aus dieser Position heraus die Vergrößerung des aktuellen durchstrahlten Messobjekts bekannt und der Einsatz der Röntgensensorik zur zweidimensionalen Messung mit Methoden der Bildverarbeitung möglich.

Der Röntgendetektor kann automatisch durch die Geräte-Software so gesteuert werden, dass der Detektor während dem eigentlichen Tomographievorgang in den Strahlkegel der Röntgenquelle positioniert und außerhalb dieser Zeiten außerhalb des Strahlungskegels in Parkstellung gebracht wird.

Durch diese Maßnahmen wird die Bestrahlungsbelastung des Röntgensensors minimiert und somit dessen Lebensdauer verlängert.

Es ist vorgesehen, dass Bildverarbeitungssensorik und Röntgensensorik eines Multisensorkoordinatenmessgerätes mit der gleichen Bildverarbeitungs-Hardware und der gleichen Bildverarbeitungs-Software bzw. Teilen davon ausgestattet sind. Dabei sind die von der Bildverarbeitungssensorik bekannten Bildbearbeitungsverfahren ebenfalls für die Röntgensensorik anwendbar.

Des Weiteren sieht die Erfindung vor, dass die 2D-Röntgenbilder vor Rekonstruktion einer Verzeichnungskorrektor und/oder einer Hellsignalkorrektur und/oder einer Dunkelsignalkorrektur und/oder einer mathematischen Translation und/oder einer mathematische Rotation und/oder einem Resamplingverfahren und/oder einer Linearitätskennlinienkorrektur und/oder einer Bildverarbeitungsfilterung unterzogen werden.

Weitere Einzelheiten, Vorteile und Merkmale der Erfindung ergeben sich nicht nur aus den Ansprüchen, den diesen zu entnehmenden Merkmalen -für sich und/oder in Kombination-, sondern auch aus der nachfolgenden Beschreibung von der Zeichnung zu entnehmenden bevorzugten Ausführungsbeispielen.

Es zeigen:

Fig. 1     eine Prinzipdarstellung eines Multisensorkoordinatenmessgerätes,
Fig. 2     ein Funktionsprinzip eines 3D-Computer-Tomographen,
Fig. 3     eine weitere Prinzipdarstellung eines Koordinatenmessgerätes,
Fig. 4     eine Prinzipdarstellung einer ersten Anordnung von Röntgenstrahlenquelle und zugeordneten Sensoren,
Fig. 5     eine Prinzipdarstellung einer zweiten Anordnung einer Röntgenstrahlenquelle und zugeordnete Sensoren,
Fig. 6.    eine Prinzipdarstellung zur Bildauswertung,
Fig. 7     eine Prinzipdarstellung zur Erläuterung eines Verfahrens zur Erhöhung der Auflösung eines Tomogramms,
Fig. 8     eine Prinzipdarstellung eines Kalibrierkörpers,
Fig. 9     eine Prinzipdarstellung eines Drehtisches mit einem Kalibrierkörper,
Fig. 10    ein Blockschaltbild und
Fig. 11    Prinzipdarstellungen zur Erläuterung eines Korrekturverfahrens.

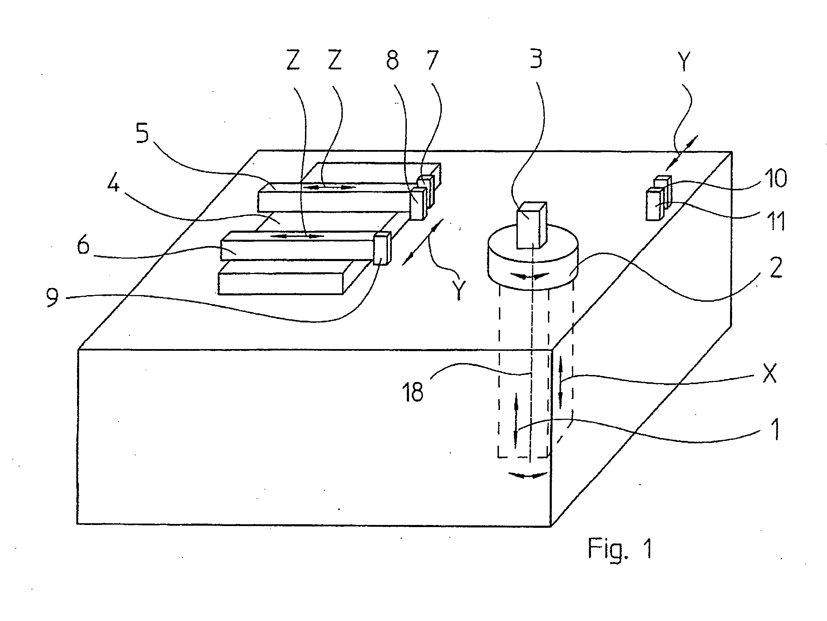

In der Fig. 1 ist prinzipiell ein Koordinatenmessgerät für den kombinierten Einsatz von Röntgensensorik und optischer und taktiler Sensorik dargestellt, gleichwenn die erfindungsgemäße Lehre in Wesentlichen Merkmalen auch für ein Koordinatenmessgerät geeignet ist, das neben dem Computer-Tomographen keine zusätzliche Sensorik umfasst.

Auf einer parallel zur X-Achse des Koordinatenmessgeräts verlaufenden Achse 18 ist ein Drehtisch 2 angeordnet. Auf diesem befindet sich ein Messobjekt 3 und kann somit um die Drehachse 18 rotiert werden und in X-Richtung durch die Achse 18 verschoben werden (Doppelpfeil). Auf einem parallel zu Y-Achse verlaufenden Schieber 4 sind zwei parallel zur Z-Achse verlaufende Achsen 5, 6 angeordnet. Auf der mechanischen Achse 5 befindet sich ein Sensor 7 für Röntgenstrahlung und ein Bildverarbeitungssensor 8. Auf der mechanischen Achse 6 befindet sich zusätzlich ein taktiler Sensor 9. Gegenüber dem Röntgensensor 7 ist eine Röntgenquelle 10 angeordnet, die wahlweise in Y-Richtung bewegbar oder fest angebracht sein kann. Gegenüber der Bildverarbeitungssensorik 8 befindet sich eine Durchlichtquelle 11. Die mechanischen Achsen bzw. Schieber, die entlang der X-, Y- bzw. Z-Achse des Koordinatenmessgerätes verlaufen, sind so ausgelegt, dass die in bzw. auf dem Koordinatenmessgerät installierten Sensoren jeweils den gesamten Messbereich auf dem Drehtisch 2 überdecken können.

Durch die Integration der Computer-Tomographie (CT) in ein Multisensor-Koordinatenmessgerät werden völlig neue Möglichkeiten geschaffen. Eine schnelle, zerstörungsfreie Komplettmessung mit der Tomographie wird mit hochgenauen Messungen von Funktionstnaßen mit taktiler oder optischer Sensorik vereint. Dabei ist erfindungsgemäß vorgesehen, dass die Röntgensensorik (Sensor, Strahlenquelle) entsprechend der zweiten Sensorik (z. B. Bildverarbeitungssensor, Durch- oder Auflichtstrahlenquelle oder taktiler Sensor gegebenenfalls mit zugeordnetem Bildverarbeitungssensor) in dem Koordinatenmessgerät positionierbar ist, dass also gleichwertig zur zweiten Sensorik die Röntgensensorik angeordnet ist. Dabei kann die Röntgensensorik mit zumindest der taktilen Sensorik und/oder der optischen Sensorik auf einer gemeinsamen mechanischen Achse angeordnet bzw. auf einer separaten mechanischen Achse angeordnet sein, die in analoger Weise wie die mechanischen Achsen für die taktile und/oder optische Sensorik arbeitet.

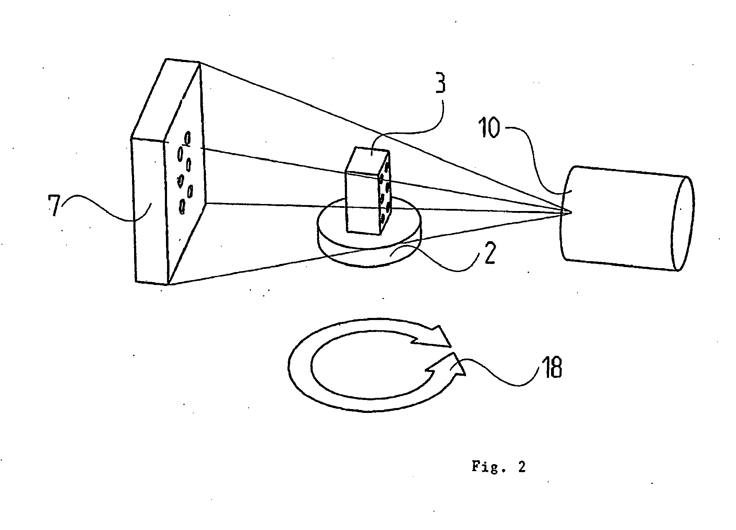

Das Funktionsprinzip der 3D-Computer-Tomographie soll anhand der Fig. 2 noch einmal erläutert werden. Dabei werden für der Fig. 1 zu entnehmenden Elemente gleiche Bezugszeichen verwendet.

Das Werkstück 3 wird auf einem Drehtisch 2 aufgelegt und mit Röntgenstrahlen durchleuchtet. Der Sensor 7 z. B. in Form eines Flächendetektors wandelt das Röntgenbild in ein digitales 2D-Bild zur weiteren Verarbeitung um. Das Objekt 3 wird um 360° gedreht und Röntgenbilder in mehreren Drehlagen aufgenommen. Anschließend erfolgt auf der Basis der 2D-Bilder eine 3D-Rekonstruktion von Messpunkten, die die gesamte zu messende Werkstückgeometrie beschreibt. Durch Integration eines oder mehrerer der weiteren Sensoren 8, 9 kann der Einsatzbereich des Computer-Tomographen erweitert werden. Mit dem Bildverarbeitungssensor 8 ist die vollautomatische Messung von komplizierten, extrem kontrastarmen Werkstücken im Durch- und Auflicht möglich. Berührende Tastsysteme ermöglichen hochgenaue Messungen von optisch nicht zugänglichen Merkmalen.

Es besteht die Möglichkeit, dass der Sensor 7 und die Röntgenstrahlenquelle 10 synchron, also bei gleich bleibendem Abstand zueinander zu dem Objekt 3 verstellt werden. Hierdurch besteht die Möglichkeit einer Messbereichsanpassung, wobei diese gegebenenfalls automatisch erfolgen kann. Alternativ kann auch das Objekt 3 zu dem Sensor 7 verstellt werden, um so eine Anpassung an die Werkstückgröße und an die Genauigkeitsanforderungen zu ermöglichen. Wird das Objekt 3 zum Sensor 7 hin verstellt, so ergibt sich eine niedrigere Vergrößeruhg, wohingegen beim Verstellen des Objektes 3 zu der Röntgenstrahlenquelle 10 hin eine hohe Vergrößerung erzielbar ist. Auch kann der Sensor bei stationärer Röntgenstrahlenquelle 10 zu dem Objekt 3 verstellt werden.

Die erfindungsgemäße Lehre bietet insbesondere nachstehende Vorteile:

- Vollständige Erfassung aller Regel- und Freiformgeometrien am Werkstück in einem Messvorgang,
- Messen von Innengeometrien und nicht zugänglichen Merkmalen (z.B. verdeckte Kanten, Hinterschneidungen),
- hochgenaue Messung von Funktionsmaßen mit taktiler oder optischer Sensorik,
- Rückführung der tomographischen Messergebnisse durch Multisensorik,
- kombiniertes Messen mit Tomographie und anderen Sensoren in einem Messablauf
- 2D- und 3D-Messung von Form, Maß und Lage,
- umfangreiche Funktionen zur 2D-Messung im Röntgenbild,
- 3D-Soll-Ist-Vergleich als 3D-Abweichungsdarstellung im Vergleich zum 3D-CAD-Modell,
- Generierung von 3D-CAD-Daten aus gewonnenen CT-Daten.

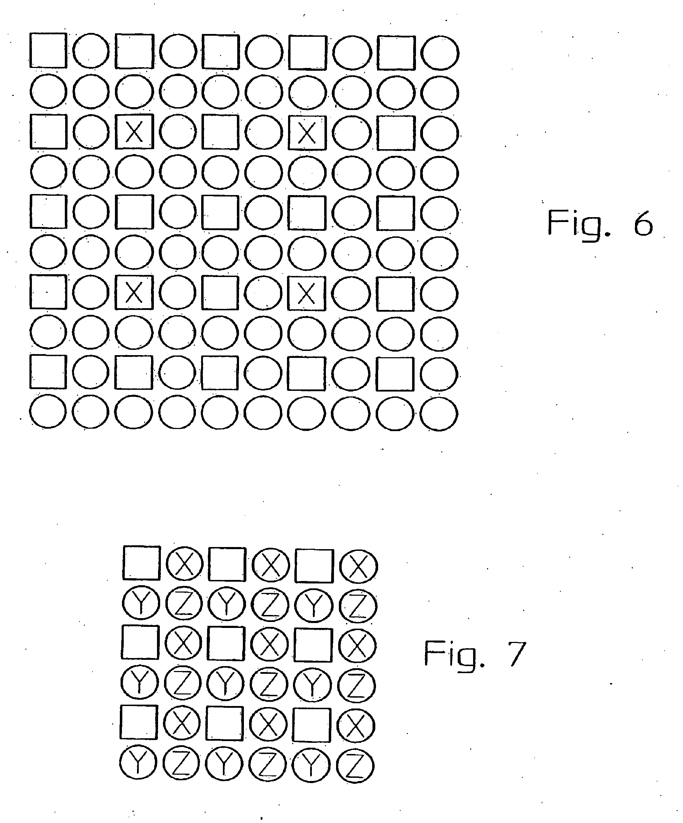

Anhand der Fig. 6 sei ein weiteres die Erfindung kennzeichnendes Verfahren erläutert, mittels dem eine Datenkompression möglich ist, ohne bezüglich der Auflösung Nachteile in Kauf nehmen zu müssen. Vielmehr besteht aufgrund der entsprechenden Lehre sogar die Möglichkeit, die Ursprungsauflösung zu übertreffen. Dies sei anhand eines 2D-Bildes erläutert.

In Fig. 6 stellen die Quadrate die Pixel eines 2D-Bildes dar. Das vorhandene 2D-Bild wird z. B. durch Mittelwertbildung aus benachbarten Pixeln in ein geringer aufgelöstes Bild mit weniger Pixelinformation (Pixel als Kreuze dargestellt) umgerechnet. Aus entsprechenden 2D-Durchstrahlungsbildern geringer Auflösung erfolgt sodann eine 3D-Rekonstruktion zur Berechnung des dreidimensionalen Voxel-Bildes. Nach Bestimmung dieses Voxel-Bildes wird das Vdxel-Bild, das in der der Fig. 6 zu entnehmenden 2D-Darstellung ebenfalls durch Kreuze simuliert ist, in ein Bild der ursprünglichen Auflösung durch Interpolation zwischen mehreren Voxel-Bilden zurückgerechnet, so dass sich - ebenfalls in 2D-Darstellung - wieder ein Bild mit Quadraten ergibt. Darüber hinaus besteht die Möglichkeit, durch weitere Anwendung der gleichen Vorgehensweise zusätzliche Voxel zu berechnen, um eine höhere Auflösung des Voxel-Bildes zu erreichen. Dies wird durch die Kreise symbolisiert.

Auf diese Weise kann schneller gerechnet werden, da eine geringere Auflösung zunächst zu Grunde gelegt werden kann, ohne im Endeffekt Einbußen in der Auflösung hinnehmen zu müssen, diese sogar übertreffen kann.

Unter Bezugnahme auf die Fig. 7 soll ein weiteres erfindungsgemäßes Verfahren veranschaulicht werden, durch das die Auflösung im Tomogramm erhöht werden kann. Hierzu werden mehrere Aufnahmen aufgenommen, zwischen denen der Sensor zu dem Objekt bzw. das Objekt zu dem Sensor um eine Strecke verschoben wird, die kleiner als Kantenlänge eines empfindlichen Elements des Sensors ist. So ist in Fig. 7 der verwendete Röntgendetektor (Sensor) in seiner Auflösung durch die als Quadrate gezeichneten Pixel gekennzeichnet. Beim Tomographieren wird in jeder Drehstellung sowohl ein Bild in der als Quadrate gezeichneten Position des Röntgendetektors aufgenommen, als auch als Bild in der als Kreise mit X gekennzeichneten Position des Röntgendetektors als auch in der als Kreise mit Y gekennzeichneten Position des Röntgendetektors als auch in der als Kreise mit Z gekennzeichneten Position des Röntgendetektors aufgenommen. Alle Bilder werden zu einem Bild zusammengefügt und als Ganzes beim Tomographie-Rekonstruktionsprozess berücksichtigt. Es wird somit eine höhere Auflösung erreicht, als durch den Detektor physikalisch gegeben ist.

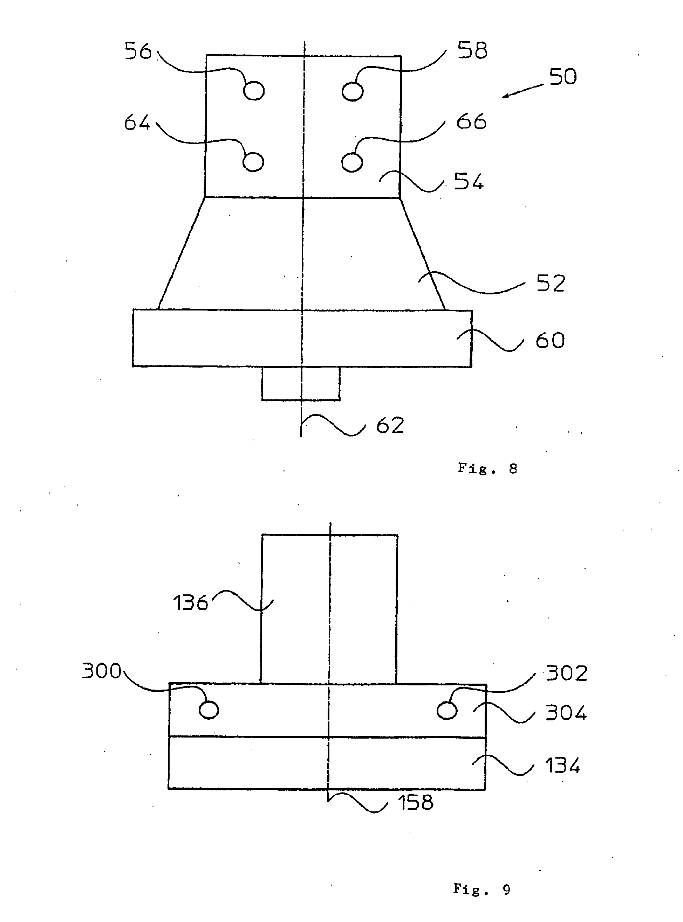

Um die Vergrößerung für die Tomographie und/oder den Drehmittelpunkt des Drehtisches 2 in Fig. 1 in Bezug auf die Röntgenquelle 10 bzw. den Sensor 7 zu bestimmen, kann ein Normal benutzt werden, das im Ausführungsbeispiel der Fig. 8 mit 50 gekennzeichnet ist. In der Prinzipdarstellung geht von einem Standfuß 52 ein Träger 54 aus einem Material aus, das eine geringe Absorption gegenüber Röntgenstrahlen aufweist. In dem Träger 54 sind zumindest zwei Kugeln 56, 58 aus Röntgenstrahlen stark absorbierendem Material wie Stahl angeordnet. Das Normal 50 wird sodann auf einen Drehtisch 60, der dem Drehtisch 2 der Fig. 1 entspricht, eines Tomographen angeordnet. Der Drehtisch 60 ist um eine Achse 62 drehbar, die mit der X-Achse des Koordinatenmessgerätes zusammenfallen kann. Die Einmessvorgänge zur Bestimmung der Lage der Drehachse 62 des Tomographen innerhalb des Koordinatenmessgerätes werden nun durch Messungen der Lagen der Kugeln 56, 58 relativ zum Röntgensensor 7 in verschiedenen Drehlagen des Kugelnormals 50 bestimmt.

Soll die Vergrößerung ermittelt werden, so sind Messungen in zwei verschiedenen Abständen zu dem Sensor 7 notwendig.

Um eine höhere Genauigkeit zu erzielen, kann das Normal 50 zwei weitere Kugeln 64, 66 aufweisen.

Nachstehend soll beschrieben werden, wie der Abstand zwischen Röntgenquelle 10 und dem Sensor 7 mittels eines Normals bestimmt wird, das im Ausführungsbeispiel aus einem Vierkugelnormal besteht, das vier auf den Ecken eines Quadrates angeordnete Kugeln umfasst.

- Die Abstände der Kugeln sind bekannt (kalibriert)
- Das Vierkugelnormal wird auf der Drehachse angeordnet
- Das Vierkugelnormal wird so eingedreht, dass die aufgespannte Ebene parallel zum Detektor steht
- Messung der vier Kugelpositionen im Bild an der Position Z1
- Berechnung der mittleren Vergrößerung M 1 aus den vier gemessenen Kugeldistanzen, den nominalen Kugeldistanzen und der nominalen Pixelgröße des Detektors
- Verfahren der Drehachse in Richtung der Quelle (oder Quelle und Detektor senkrecht zur Drehachse)
- Messung der vier Kugelpositionen im Bild an der Position Z2
- Berechnung der mittleren Vergrößerung M2 aus den vier gemessenen Kugeldistanzen, den nominalen Kugeldistanzen und der nominalen Pixelgröße des Detektors
- Berechnung des Abstands Quelle Detektor nach folgender Gleichung:

$$AQD = dZ * M1 * M2 / ( M2 - M2 )$$

darin ist:

AQD: Abstand Quelle Detektor
M1: Vergrößerung an der Position Z1
M2: Vergrößerung an der Position Z2
dZ: Distanz zwischen den Position Z1 und 22

- Berechnung der Distanz Quelle zu Z1 aus folgender Gleichung:

$$D1 = dZ * M2 / ( M1 - M2 )$$

- Berechnung der Distanz Quelle zu Z2 aus folgender Gleichung:

$$D2 = D1 + dZ = dZ * M1 / ( M1 + M2 )$$

- Berechnung der Position der Kegelachse auf dem Detektor nach folgender Gleichung

$$Pd = (Pkn1*D1 - Pkn2*D2) / dZ$$

darin ist:

Pd: Abweichungsvektor der Kegelachs-Position von der Detektormitte
Pkn1: Positionsvektor der Kugel n auf dem Detektor an der Position Z1
Pkn2: Positionsvektor der Kugel n auf dem Detektor an der Position Z2

- Berechnung des mittleren Abweichungsvektors aus den vier Abweichungsvektoren für jede Kugelposition

Ein Verfahren zur Bestimmung der Y-Position des Drehachsenmittelpunktes unter Zugrundelegung ebenfalls eines Vierkugel-Normals mit an den Ecken eines Quadrates angeordneten Kugeln erfolgt wie nachstehend:

- Die Abstände der Kugeln sind bekannt (kalibriert)
- Das Vierkugelnormal wird auf der Drehachse angeordnet
- Das Vierkugelnormal wird so eingedreht, dass die aufgespannte Ebene parallel zum Detektor steht

- Messung der vier Kugelpositionen im Bild
- Berechnung der mittleren Vergrößerung M 1 aus den vier gemessenen Kugeldistanzen, den nominalen Kugeldistanzen und der nominalen Pixelgröße des Detektors
- Drehung der Drehachse um 180°
- Messung der vier Kugelpositionen im Bild
- Berechnung der mittleren Vergrößerung M2 aus den vier gemessenen Kugeldistanzen, den nominalen Kugeldistanzen und der nominalen Pixelgröße des Detektors,
- Berechnung der Y Pösition des Drehmittelpunktes anhand der vier Kugelpositionen vor und nach der Drehung nach folgender Gleichung:

$$Pdyn = (Pkyn1 * M2 + Pkyn2 * M1) / (M1*M2)$$

darin ist:

Pdyn: Y Position der Drehachse auf dem Detektor für Kugel n
Pkyn1: Y Position der Kugel n bei Drehwinkel 0°
Pkyn2: Y Position der Kugel n bei Drehwinkel 180°
M1: mittlere Vergrößerung bei Drehwinkel 0°
M2: mittlere Vergrößerung bei Drehwinkel 180°.

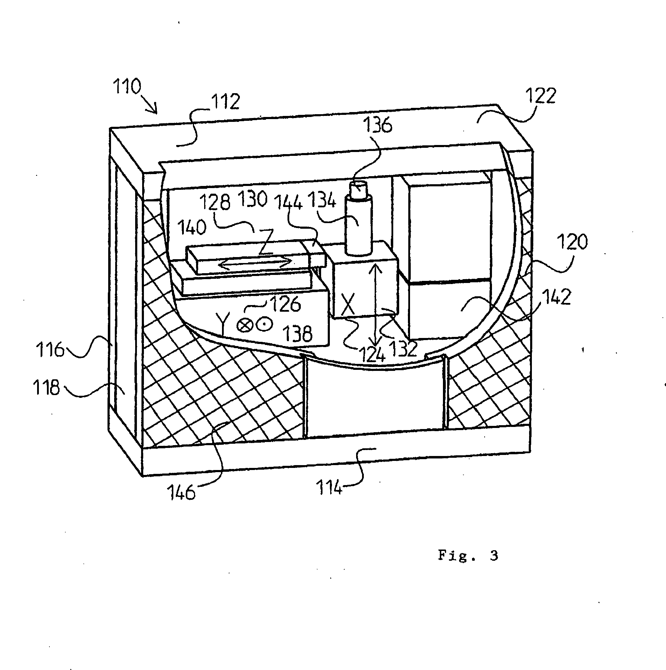

Den Fig. 3 bis 5 sind weitere Merkmale der erfindungsgemäßen Lehre zu entnehmen. Dabei ist in Fig. 3 ebenfalls rein prinzipiell ein Koordinatenmessgerät 110 mit einem Gehäuse 112 dargestellt, das eine Grundplatte 114, Rückwandung 116, Seitenwandungen 118, 120 sowie eine Kopfwandung 122 umfasst, die auch als Deckplatte zu bezeichnen ist.

Die x-Achse, y-Achse und z-Achse des Koordinatenmessgerätes sind in der Zeichnung mit dem Bezugszeichen 124, 126 und 128 gekennzeichnet. An der Innenseite 130 der Rückwandung 116 des Gehäuses 112 verläuft in x-Richtung einer Führung, entlang der, also in x-Richtung 124, eine Halterung 132 für einen Drehtisch 134 verstellbar ist, auf dem ein zu messendes Objekt 136 angeordnet ist. Mit anderen Worten ist auf der x-Achse 124 der Drehtisch 134 angeordnet.

Entlang der y-Achse 126 verlaufen Führungen, entlang der eine Aufnahme 138 verschiebbar ist. Von der Aufnahme 138 geht entlang der z-Achse 128 verschiebbar eine Halterung 140 aus.

Von der Grundplatte 114 geht des Weiteren eine Röntgenquelle 142 aus, deren Röntgenstrahlung das auf dem Drehtisch 134 angeordnete Objekt 136 durchsetzen. Die Röntgenstrahlung wird ihrerseits von entsprechend geeigneten Sensoren wie CCD-Sensoren erfasst, die für Röntgenstrahlung empfindlich sind.

Des Weiteren können von der z-Achse 128, d. h. im Ausführungsbeispiel von der Halterung 140 Sensoren 144 ausgehen. Hierbei kann es sich um Sensoren handeln, die für Koordinatenmessgeräte üblich sind, also z. B. taktile oder optische Sensoren. Somit kann sowohl tomographiert werden als auch taktil oder optisch wie mit Bildverarbeitungssensoren, Laserabstandssensoren etc. gemessen werden.

Durch den Einsatz von Röntgenstrahlen ist es erforderlich, dass das Koordinatenmessgerät 110 im hinreichenden Umfang nach außen hin abgeschirmt ist. Hierzu ist erfindungsgemäß vorgesehen, dass zumindest einige der tragenden Bauelemente Abschirmfunktion ausüben. So kann z. B. die Grundplatte 114 und/oder die Rückwand 116 derart dimensioniert bzw. ausgebildet sein, dass die erforderliche Abschirmfunktion sichergestellt ist.

Die entsprechenden Wandungen 114, 116 üben dabei gleichzeitig die Funktion aus, die für den messtechnischen Aufbau erforderlich ist, nämlich im Ausführungsbeispiel Führung für die x- und y-Achse.

Zusätzlich besteht die Möglichkeit, Wandungen, die nicht die hinreichende Abschirmwirkung aufweisen, mit strahlungsdämmenden Schichten 146 innen- und/oder außenflächenseitig zu versehen. Hierbei handelt es sich insbesondere um Bleiblech.

Bezüglich der tragenden Wandungen, insbesondere derjenigen, die Abschirmfunktion ausüben, sollten bevorzugterweise Hartgestein wie Granit oder entsprechende Materialien zum Einsatz gelangen. Auch ist ein künstliches Hartgestein wie Polymerbeton denkbar, das im erforderlichen Umfang mit Röntgenstrahlen absorbierendem Material wie Magneteisenstein oder ähnliches versetzt sein kann.

Erfindungsgemäß übt das Gehäuse 112 des Koordinatenmessgerätes 110 bzw. Teile dieses eine Doppelfunktion aus, nämlich die der erforderlichen Abschirmung sowie die von Funktionsbauteilen des messtechnischen Aufbaus. Somit ergibt sich eine kompakte Konstruktion.

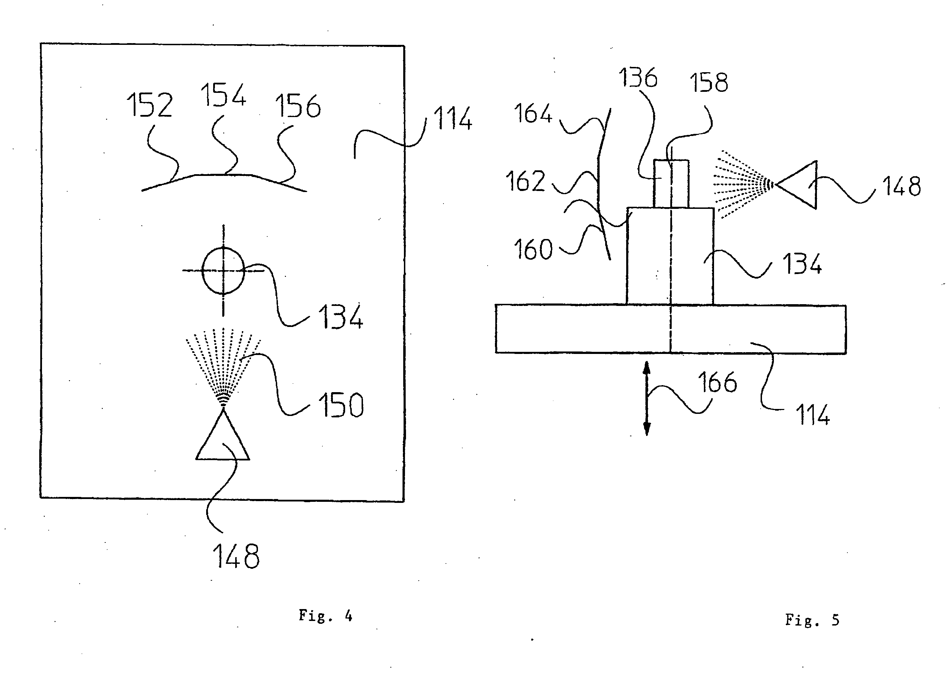

Um eine hohe Messdichte zu erreichen bzw. in jeweiliger Messposition nur geringe Bestrahlungszeiten in Kauf nehmen zu müssen, ohne dass Einbußen hinsichtlich der Messgenauigkeit erfolgen, ist entsprechend der Darstellung der Fig. 4 vorgesehen, dass gleichzeitig - also in jeder Messposition des Objekts 136 - mehrere Tomogramme unter unterschiedlichen Durchstrahlungswinkeln aufgenommen wird. So geht in Fig. 4 entsprechend den Ausführungen gemäß Fig. 3 von der Grundplatte 114 der Drehtisch 134 aus, auf dem ein nicht dargestelltes zu messendes Objekt angeordnet ist, das von von einem Röntgenstrahler 148 stammender Röntgenstrahlung 150 durchstrahlt wird. Die Strahlung wird im Ausführungsbeispiel von insgesamt drei Röntgensensoren 152, 154, 156 erfasst, so dass sich drei Tomogramme für unterschiedliche Durchstrahlungsrichtungen in einer Messposition des Objektes ergeben. In jeder Messposition, also jeder Winkelstellung des Drehtisches 34 werden die Sensoren 152, 154, 156 ausgelesen und Projektionsbilder für das Tomogramm gewonnen. Dabei ist die Winkelstellung der Sensoren 152, 154, 156 derart ausgelegt, dass sich die Winkel zwischen den Sensoren 152, 154, 156 jeweils um ganzzahlige Vielfache des beim Betreiben des Computer-Tomographen verwendeten Winkelschritts des Drehtisches 136 unterscheiden, wobei der zweite und der dritte Sensor 154, 156 zum vorausgehenden ersten Sensor 152 bzw. zweiten Sensor 154 um ein Drittel des Winkelschritts verdreht angeordnet sind.

Um mehrere Tomogramme des zu messenden Objekts 136 aufzunehmen, wobei der Winkel zwischen Drehachse 158 des Drehtisches 154 und Röntgenstrahlung 150 scheinbar verändert wird, sind im Ausführungsbeispiel der Fig. 5 beispielhaft drei Sensoren 160, 162, 164 in unterschiedlichen Winkeln zu der Hauptstrahlungsrichtung der Röntgenstrahlungsquelle 148 angeordnet, wodurch das scheinbare Verschwenken der Röntgenstrahlungsquelle zur Drehachse 158 simuliert wird.

Der in Fig. 5 eingezeichnete Doppelpfeil 166 soll symbolisieren, dass der Drehtisch 134 entlang der Drehachse 158 parallel zur X-Achse verstellbar ist.

Wie anhand der Fig. 9 prinzipiell verdeutlicht wird, kann beim Tomographieren grundsätzlich ein Kalibrierkörper vorzugsweise in Form von Kugeln 300, 302 mit tomographiert werden, wodurch sich die Relativlage der Drehachse 158 des Drehtisches 134 ergibt, auf dem das zu messende Objekt 136 angeordnet ist. Die Kugeln 300, 302 können in einer Aufnahme 304 angeordnet sein, die gegenüber Röntgenstrahlen eine geringe Absorption zeigt, wohingegen die Kugeln 300, 302 stark absorbierend sind und z. B. aus Stahl bestehen. Hierdurch kann problemlos während des Tomographierens die Position der Drehachse 158 zum Koordinatenmessgerät bzw. zur Röntgenquelle 10 bzw. zum Sensor 7 bestimmt und anschließend mathematisch korrigiert werden.

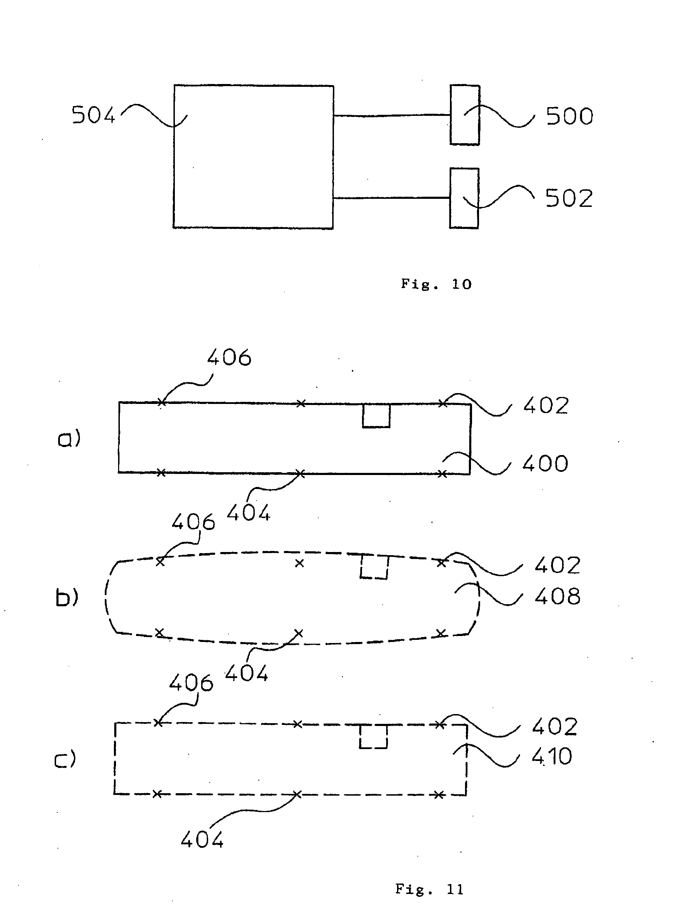

Erfindungsgemäß werden in dem Koordinatenmessgerät mittels taktiler und/oder optischer Sensorik Messpunkte am Messobjekt aufgenommen und zur Korrektur der mit der Röntgensensorik ermittelten Messpunkte herangezogen. Dies soll auch anhand der Fig. 11 verdeutlicht werden. Dieser ist das Prinzip eines entsprechenden Korrekturverfahrens zu entnehmen. So ist in Fig. 11a ein Messobjekt 400 dargestellt, das an ausgewählten Punkten taktil und/oder optisch gemessen wird. Entsprechende Messpunkte sind beispielhaft mit den Bezugzahlen 402, 404, 406 gekennzeichnet. Beim Tomographieren, das anschließend im selben Koordinatenmessgerät stattfindet, erhält man die durch typische Fehler der Tomographie veränderte Gestalt in der tomographierten Punktwolke 408. Dies können z. B. tomographietypische Artefakte sein. Die tomographierten Messpunkte werden auf der Basis der zur Verfügung stehenden mit optischer und/oder taktiler Sensorik genauer gemessenen Messpunkte, die in Fig. 11b noch einmal eingezeichnet sind, in ihrer Lage korrigiert. Dabei kann zwischen den taktilen und optisch gemessenen Messpunkten eine Interpolation erfolgt sein. Als Ergebnis erhält man sodann eine geometrisch korrigierte tomographisch gemessene Punktwolke 410, die besser der Gestalt des Messobjekts 400 entspricht als die Originaldaten des Tomogramms. Dies zeigt ein Vergleich der Fig. 11b und 11c.

Bei der Durchführung der Messungen und der Auswertung der Messergebnisse kann die Bildverarbeitungssensorik für das Messen mit sichtbarem Licht im Durchlichtverfahren - gegebenenfalls auch im Auflichtverfahren - mit der gleichen Bildverarbeitungsauswerteeinheit bzw. dem gleichen Bildverarbeitungsboard wie die Röntgensensorik gekoppelt werden. Softwaregesteuert kann sodann zwischen den beiden Sensoren umgeschaltet und in der gleichen Hardware digitalisiert und gerechnet werden. Dies wird prinzipiell durch die Fig. 10 verdeutlicht, in der eine Bildverarbeitungssensorik 500 und eine Röntgensensorik 502 mit dem gleichen Bildverarbeitungsboard 504 verbunden sind, um in zuvor beschriebener Weise arbeiten zu können.

**Patentansprüche**

1.  Verfahren zum Messen eines Objekts mit einem Koordinatenmessgerät enthaltend zumindest einen Computer-Tomographen mit Röntgenquelle und Röntgendetektor,
    **dadurch gekennzeichnet,**
    **dass** die Röntgenquelle entlang einer Achse des Koordinatenmessgerätes verfahrbar oder fest angeordnet ist, der Röntgendetektor entlang einer Achse des Koordinatenmessgerätes verfahrbar angeordnet ist und dass alle Einstellparameter, die für das Tomographieren mit einer bestimmten Vergrößerung oder in einem bestimmten Messbereich notwendig sind, nämlich die Position für Röntgenquelle und Röntgendetektor, die Position der verschiedenen Achsen des Tomographie- bzw. Koordinatenmessgerätes, die diesen Positionen zugeordneten Vergrößerungswerte, sowie Korrekturwerte zur Position der Achsen bezüglich des durch die zugeordneten Wegmesssysteme gelieferten Positionsmesswertes, in einem einmaligen Einmessvorgang des Koordinatenmessgerätes messtechnisch erfasst und abgespeichert werden und die so gespeicherten Daten ohne weitere Einmessvorgänge für nachfolgende Messungen mit dem Computer-Tomographen benutzt werden.

2. Verfahren nach Anspruch 1,
**dadurch gekennzeichnet,**
**dass** vorab eingemessene Vergrößerungs- und Messbereichseinstellungen durch das Messprogramm des Koordinatenmessgerätes automatisch aufgerufen und die entsprechenden Hardware-Komponenten des Gerätes positioniert werden.

3. Verfahren nach Anspruch 1 oder 2,
**dadurch gekennzeichnet,**
**dass** die Röntgenquelle und der Röntgendetektor synchron verfahren werden, um lediglich die Vergrößerung und / oder den Messbereich zu verändern.

4. Verfahren nach Anspruch 1 oder 2,
**dadurch gekennzeichnet,**
**dass** die Röntgenquelle und der Röntgendetektor unabhängig voneinander verfahren werden, um die Vergrößerung und / oder den Messbereich zu verändern.

5. Verfahren nach einem der Ansprüche 1 bis 4,
**dadurch gekennzeichnet,**
**dass** alle für das Tomographieren notwendigen Einstellungen im Vorhinein eingemessen und abgespeichert werden und beim jeweiligen Tomographiervorgang keinerlei Einmessvorgänge mehr notwendig sind.

6. Verfahren nach einem der Ansprüche 1 bis 5,
**dadurch gekennzeichnet,**
**dass** die Justage für den Drehmittelpunkt des auf einem Drehtisch angeordneten Objekts durch einen Einmessvorgang und / oder eine entsprechende Korrektur des Drehmittelpunktversatzes in der Software realisiert wird.

7. Verfahren nach Anspruch 6,
**dadurch gekennzeichnet,**
**dass** die Bestimmung der Vergrößerung für die Tomographie und / oder die Bestimmung der Drehmittelpunktlage in Bezug auf die Röntgenquelle und den Röntgendetektor mittels eines Normals bestimmt wird, das aus mindestens zwei Kugeln besteht.

8. Verfahren nach Anspruch 6,
**dadurch gekennzeichnet,**
**dass** die Bestimmung der Vergrößerung für die Tomographie und/oder die Bestimmung der Drehmittelpunktlage in Bezug auf die Röntgenquelle und den Röntgendetektor mittels eines Normals bestimmt wird, das aus vier Kugeln besteht.

9. Verfahren nach Anspruch 8,
**gekennzeichnet durch** die folgenden Verfahrensschritte zur Bestimmung der Position des Drehachsmittelpunktes im Koordinatenmessgerät:

- ein Vierkugelnormal bestehend aus vier in den Ecken eines Rechtecks wie Quadrats angeordneten Kugeln, bei dem die Abstände der Kugeln zueinander bekannt bzw. kalibriert sind, wird auf der Drehachse positioniert,
- das Vierkugelnormal wird so verdreht, dass die aufgespannte Ebene parallel zum Detektor steht,
- Messung der Vierkugelposition im Messfeld des Detektors,
- Berechnung der mittleren Vergrößerung M1 aus den vier gemessenen Kugeldistanzen, den nominalen Kugeldistanzen und der nominalen Pixelgröße des Detektors,
- Drehung der Drehachse um 180°,
- Messung der vier Kugelpositionen im Bild,
- Berechnung der mittleren Vergrößerung M2 aus den vier gemessenen Kugeldistanzen, den nominalen Kugeldistanzen und der nominalen Pixelgröße des Detektors.

10. Verfahren nach Anspruch 9,

**dadurch gekennzeichnet,**
**dass** die Berechnung der Y-Position des Drehmittelpunktes anhand der vier Kugelpositionen vor und nach der Drehung erfolgt nach folgender Gleichung: Pdyn = (Pkyn1 * M2 + Pkyn2 + M1) / (M1*M2) mit Pdyn: Y-Position der Drehachse auf dem Detektor für Kugel n, Pkyn1: Y-Position der Kugel n bei Drehwinkel 0°, Pkyn2: Y-Position der Kugel n bei Drehwinkel 180°, M1: mittlere Vergrößerung bei Drehwinkel 0°, M2: mittlere Vergrößerung bei Drehwinkel 180°.

11. Verfahren nach einem der Ansprüche 1 bis 10,
**dadurch gekennzeichnet,**
**dass** in dem Koordinatenmessgerät mittels taktiler und / oder optischer Sensorik am Messobjekt gemessene Messpunkte zur Korrektur der mit dem Computer-Tomographen ermittelten Messpunkte berücksichtigt werden.

12. Verfahren nach Anspruch 11,
**dadurch gekennzeichnet,**
**dass** eine Korrektur der mit der Tomographie gemessenen Messpunktewolke des Messobjektes oder der hieraus errechneten triangulierten Flächenelemente durch taktil und / oder optisch gewonnene Messpunkte erfolgt.

13. Verfahren nach Anspruch 12,
**dadurch gekennzeichnet,**
**dass** zwischen den taktil und / oder optisch gemessenen Korrekturpunkten interpoliert wird.

14. Verfahren nach Anspruch 13,
**dadurch gekennzeichnet,**
**dass** zwischen den mit taktil und / oder optischer Sensorik gewonnenen Korrekturpunkten unter Berücksichtigung des Funktionsverlaufes der durch die Tomographie gemessenen Punktwolke interpoliert wird und / oder durch Berücksichtigung eines Nominal-CAD-Modells interpoliert wird.

15. Verfahren nach einem der Ansprüche 1 bis 14,
**dadurch gekennzeichnet,**
**dass** ein kalibriertes Teil eines Messobjekttyps tomographiert wird und aus der Messabweichung bei der Messung ein Korrekturnetzwerk für die Korrektur der tomographischen Messwerte errechnet wird und beim Messen von Serienteilen die tomographischen Messungen mit den vorher abgestimmten Korrekturwerten abgestimmt werden.

16. Verfahren nach einem der Ansprüche 11 bis 14,
**dadurch gekennzeichnet,**
**dass** zuerst ein Musterteil eines Messobjekttyps tomographisch und taktil und /oder optisch abgetastet wird, aus der Differenz beider Messungen ein Korrektumetzwerk für die Korrektur der tomographischen Messwerte errechnet wird und beim Messen von Serienteilen die tomographischen Messungen mit den einmalig bestimmten Korrekturwerten korrigiert werden.

17. Verfahren nach Anspruch 15 oder 16,
**dadurch gekennzeichnet,**
**dass** bei der Messung von Serienteilen zusätzlich einzelne optisch und / oder taktil gemessene Korrekturpunkte berücksichtigt werden.

18. Verfahren nacht Anspruch 16 oder 17,
**dadurch gekennzeichnet,**
**dass** die taktilen und / oder optischen Messpunkte zur Korrektur durch einen Bediener grafisch an der durch Tomographie bestimmten Punktwolke festgelegt werden und durch das Koordinatenmessgerät dann automatisch gemessen werden.

19. Verfahren nach Anspruch 16 oder 17,
**dadurch gekennzeichnet,**
**dass** die taktilen und / oder optischen Messpunkte zur Korrektur durch einen Bediener grafisch an dem CAD-Modell des zu messenden Teiles festgelegt werden und durch das Koordinatenmessgerät dann automatisch gemessen werden.

20. Verfahren nach Anspruch 19,

**dadurch gekennzeichnet**

**dass** die taktilen und / oder optischen Messpunkte zur Korrektur durch einen automatischen Algorithmus auf der Oberfläche des CAD-Modells annähernd gleichmäßig oder gleichmäßig verteilt und durch das Koordinatenmessgerät automatisch gemessen werden.

21. Verfahren nach Anspruch 19 oder 20,
**dadurch gekennzeichnet,**
**dass** die taktilen und / oder optischen Messpunkte zur Korrektur durch einen Bediener am CAD-Modell im Vornhinein festgelegt werden und nach dem Laden des CAD-Modells durch das Koordinatenmessgerät automatisch gemessen werden.

22. Verfahren nach einem der Ansprüche 6 bis 21,
**dadurch gekennzeichnet,**
**dass** beim Tomographiervorgang grundsätzlich ein Kalibrierkörper, insbesondere eine Anordnung von Kugeln, mittomographiert wird und hieraus die Relativlage der Drehachse zum Koordinatenmessgerät und / oder zur Röntgenquelle und / oder zum Röntgensensor bestimmt und die wirksame Vergrößerung anschließend mathematisch korrigiert wird.

23. Verfahren nach Anspruch 22,
**dadurch gekennzeichnet,**
**dass** der Kalibrierkörper, insbesondere die Kalibrierkugeln, in einem Träger mit geringeren Röntgenabsorptionseigenschaften als der Kalibrierkörper untergebracht wird und unter Berücksichtigung des Kalibrierkörpers das Messobjekt auf dem Drehtisch positioniert wird.

24. Verfahren nach Anspruch 22 oder 23,
**dadurch gekennzeichnet,**
**dass** die Lage des Kalibrierkörpers auf der Drehachse mit optischen und / oder taktilen Sensoren bestimmt und zur Korrektur der Lage der Drehachse herangezogen werden.

25. Verfahren nach einem der Ansprüche 22 bis 24,
**dadurch gekennzeichnet,**
**dass** die Raumlage der Drehachse in Bezug zur Röntgenquelle und zum Röntgendetektor mit dem Computer-Tomographen und / oder mit der taktilen Sensorik und /oder mit der optischen Sensorik messtechnisch bestimmt wird und diese Lageabweichung beim Tomographieren von Messobjekten mathematisch korrigiert wird.

26. Verfahren nach einem der Ansprüche 6 bis 25,
**dadurch gekennzeichnet,**
**dass** die von der Nominallage abweichende Lage der Drehachse durch Rotation und / oder Translation und/oder Verzerrung der 2D-Einzelbilder korrigiert wird.

27. Verfahren nach zumindest einem der Ansprüche 6 bis 26,
**dadurch gekennzeichnet,**
**dass** die von der Nominallage abweichende Lage der Drehachse in einem Rekonstruktionsalgorithmus berücksichtigt wird.

28. Verfahren nach einem der Ansprüche 6 bis 27,
**dadurch gekennzeichnet,**
**dass** das Messobjekt durch den Einsatz von taktilen und / oder optischen Sensoren und / oder von Tomographie in seiner Lage auf dem Drehtisch des Messgerätes und somit im Gerätekoordinatensystem bestimmt wird und anschließend im 2D-Durchstrahlungsmodus an eingemessener Position des Röntgensensors mit Methoden der Bildverarbeitung gemessen wird.

29. Verfahren nach einem der Ansprüche 1 bis 28,
**dadurch gekennzeichnet,**
**dass** der Computer-Tomograph automatisch durch die Geräte-Software gesteuert wird, wobei der Röntgensensor während des Tomographievorgangs in den Strahlkegel der Röntgenquelle positioniert wird und außerhalb dieser Zeiten außerhalb des Strahlungskegels in Parkstellung gebracht wird.

**30.** Verfahren nach einem der Ansprüche 1 bis 29,
**dadurch gekennzeichnet,**
**dass** eine Bildverarbeitungssensorik als die zweite Sensorik und der Computer-Tomograph des Koordinatenmessgeräts in Form eines Multisensorkoordinatenmessgerätes mit der gleichen Bildverarbeitungshardware und der gleichen Bildverarbeitungssoftware bzw. Teilen davon ausgestattet sind.

**31.** Verfahren nach Anspruch 30,
**dadurch gekennzeichnet,**
**dass** die von der Bildverarbeitungssensorik bekannten Bildbearbeitungsverfahren ebenfalls für den Computer-Tomographen anwendbar sind.


**Claims**

**1.** Method to measure an object with a coordinate measuring device comprising at least a computer tomography with an x-ray source and an x-ray detector,
**characterized in that**
the x-ray source is arranged along an axis of the coordinate measuring device in a movable or stationary manner, the x-ray detector is arranged along an axis of the coordinate measuring device in a movable manner, and that all adjustment parameters necessary for the tomography with a particular magnification or within a particular measuring range, namely the position for x-ray source and x-ray detector, the position of the different axes of the tomography device and the coordinate measuring device, respectively, the magnification values assigned to these positions as well as correction values for the position of the axes in relation to the measured position value provided by the assigned position measuring systems, are recorded by using measurement technique and stored during a one-time calibration process of the coordinate measuring device, and the thus stored data are being used without any further calibration processes for the following measurements conducted with the computer tomography.

**2.** The method according to claim 1,
**characterized in that**
previously calibrated magnification and measurement range settings are automatically called up by the measurement program of the coordinate measuring device, and the corresponding hardware components of the device are positioned.

**3.** The method according to claim 1 or 2,
**characterized in that**
the x-ray source and the x-ray detector are synchronously driven in order to change only the magnification and/or measurement range.

**4.** The method according to claim 1 or 2,
**characterized in that**
the x-ray source and the x-ray detector are driven independently of one another to change the magnification and/or measurement range.

**5.** The method according to one of the claims 1 to 4,
**characterized in that**
all settings necessary for the tomography are calibrated and stored in advance and that for each tomography procedure, calibration procedures are no longer necessary.

**6.** The method according to one of the claims 1 to 5
**characterized in that**
the adjustment for the rotation center of the object arranged on a rotating table is realized through a calibration procedure and/or a corresponding correction of the rotation center drift in the software.

**7.** The method according to claim 6,
**characterized in that**
the magnification for the tomography and/or the position of the rotation center in relation to the x-ray source and x-ray detector is determined using a standard that consists of at least two spheres.

**8.** The method according to claim 6,
**characterized in that**
the magnification for the tomography and/or the position of the rotation center in relation to the x-ray source and x-ray detector is determined using a standard that consists of at least four spheres.

**9.** The method according to claim 8,
**characterized by** the following method steps for determining the position of the rotation center in the coordinate measuring device:

- a four-sphere standard consisting of four spheres arranged at the corners of a rectangle such as a square, wherein the spacing of the spheres in relation to one another is known and calibrated, respectively, is positioned on the rotation axis,
- the four-sphere standard is rotated so that the defined plane is parallel to the detector,
- measurement of the four-sphere position in the measuring field of the detector,
- calculation of the average magnification M1 from the four measured sphere distances, the nominal sphere distances and the nominal pixel size of the detector,
- rotation of the rotation axis by 180°,
- measurement of the four sphere positions in the image,
- calculation of the average magnification M2 from the four measured sphere distances, the nominal sphere distances and the nominal pixel size of the detector.

**10.** The method according to claim 9,
**characterized in that**
the Y-position of the rotation center is calculated from the four sphere positions prior to and following the rotation using the following formula: Pdyn = (Pkyn1 * M2 + Pkyn2 + M1) / (M1 * M2) with Pdyn being the Y-position of the rotation axis on the detector for sphere n, Pkyn1 being the Y-position of the sphere n at a rotation angle 0°, Pkyn2 being the Y-position of the sphere n at a rotation angle 180°, M1 being the average magnification at a rotation angle 0° and M2 being the average magnification at a rotation angle 180°.

**11.** The method according to one of the claims 1 to 10,
**characterized in that**
in the coordinate measuring device, the measurement points measured by tactile and/or optic sensor systems on the measuring object are taken into consideration for the correction of the measurement points determined by the tomography.

**12.** The method according to claim 11,
**characterized in that**
that the measurement point cloud of the object of measurement measured with the tomography or the triangulated surface element calculated from this data is corrected through tactilely and/or optically obtained measurement points.

**13.** The method according to claim 12,
**characterized in that**
it is interpolated between the tactilely and/or optically measured correction points.

**14.** The method according to claim 13,
**characterized in that**
it is interpolated between correction points obtained with a tactile and/or optical sensor system while factoring in the functional course of the point cloud measured through the tomography, and/or by taking into consideration the nominal CAD-model.

**15.** The method according to one of the claims 1 to 14,
**characterized in that**
a calibrated portion of a type of a measurement object undergoes tomography, that a correction network for the correction of tomographic measurement values is calculated from the measurement deviation occurring during measurement and, when repetition parts are measured, the tomographic measurements are adjusted with the previously calibrated correction values.

**16.** The method according to one of the claims 11 to 14,

**EP 1 749 190 B1**

**characterized in that**
at first a sample part of the type of object of measurement is scanned tomographically as well as tactilely and/or optically, a correction network for the correction of the tomographic measurement values is calculated from the difference of both measurements and, when repetition parts are measured, the tomographic measurements are corrected with the one-time defined correction values.

17. The method according to claim 15 or 16,
**characterized in that**
when repetition parts are measured, individual optically and/or tactilely measured correction points are additionally factored in.

18. The method according to claim 16 or 17,
**characterized in that**
for correction, the tactile and/or optical measurement points are graphically plotted by an operator on the point cloud generated by tomography and are then measured automatically by the coordinate measuring device.

19. The method according to claim 16 or 17,
**characterized in that**
for correction, the tactile and/or optical measurement points are graphically plotted by an operator on the CAD-model of the part to be measured and are then automatically measured by the coordinate measuring device.

20. The method according to claim 19,
**characterized in that**
the tactile and/or optical measurement points for correction are nearly evenly or evenly distributed on the surface of the CAD-model by an automatic algorithm and are automatically measured by the coordinate measuring device.

21. The method according to claim 19 or 20,
**characterized in that**
the tactile and/or optical measurement points for correction are predefined on the CAD-model by an operator and, after the CAD-model has been loaded, are automatically measured by the coordinate measuring device.

22. The method according to one of the claims 6 to 21
**characterized in that**
basically, when a tomography procedure is performed, a calibration body, in particular, an arrangement of spheres, is also subjected to tomography, thereby determining the relative position of the rotation axis in relation to the coordinate measuring device and/or to the x-ray source and/or to the x-ray sensor and the effective magnification is determined and then mathematically corrected.

23. The method according to claim 22,
**characterized in that**
the calibration body, in particular the calibration spheres, is placed in a carrier with lower radiation absorption properties than that of the calibration body, while the object of measurement is positioned on the rotating table, with the calibration body being taken into consideration.

24. The method according to claim 22 or 23,
**characterized in that**
the position of the calibration bodies on the rotation axis is determined with optical and/or tactile sensors and used for the correction of the position of the rotation axis.

25. The method according to claim 22 or 24,
**characterized in that**
the spatial location of the rotation axis in relation to the x-ray source and x-ray detector is measurement-technically ascertained with the tomography and/or with the tactile sensor system and/or with the optical sensor system, and this deviation in position is mathematically corrected when objects of measurement undergo tomography.

26. The method according to one of the claims 6 to 25,
**characterized in that**
the position of the rotation axis deviating from the nominal position is corrected through rotation and/or translation and/or distortion of the 2D-single images.

27. The method according one of the claims 6 to 26,
**characterized in that**
the position of the rotation axis deviating from the nominal position is factored into the reconstruction algorithm.

28. The method according to one of the claims 6 to 27,
**characterized in that**
the position of the object of measurement on the rotating table and thus in the machine coordinate system is ascertained using tactile and/or optical sensors and/or tomography and then measured in 2D x-ray mode at calibrated position of the x-ray sensor using methods of image processing.

29. The method according to one of the claims 1 to 28,
**characterized in that**
the computer tomography is automatically controlled via the device-software, while the x-ray sensor is positioned in the radiation cone of the x-ray source during the tomography procedure and, at other times, is brought into parked position outside the radiation cone.

30. The method according to one of the claims 1 to 29,
**characterized in that**
an image-processing sensor system as the second sensor system and the computer tomograph of the coordinate measuring device in the form of a multisensor coordinate measuring device are equipped with the same image-processing hardware and the same image-processing software and parts thereof, respectively.

31. The method according to claim 30,
**characterized in that**
the image processing methods known from image-processing sensor technology can also be used for the tomography.

## Revendications

1. Procédé destiné à mesurer un objet avec un appareil de mesure de coordonnées, comprenant au moins un tomodensitomètre avec une source de rayons X et un détecteur de rayons X,
**caractérisé en ce**
**que** la source de rayons X est disposée le long d'un axe de l'appareil de mesure de coordonnées de manière mobile ou fixe, que le détecteur de rayons X est disposé le long d'un axe de l'appareil de mesure de coordonnées de manière mobile et que tous les paramètres de réglage requis pour la tomographie avec un certain grossissement ou dans une certaine plage de mesure, à savoir le positionnement de la source de rayons X et du détecteur de rayons X, le positionnement des différents axes du tomodensitomètre et/ou de l'appareil de mesure de coordonnées, les valeurs de grossissement affectées à ces positionnements, ainsi que des valeurs correctives relatives au positionnement des axes par rapport à la valeur de positionnement mesurée fournie par les systèmes de mesure de déplacement associés, sont métrologiquement saisis et enregistrés lors d'une unique opération de calibrage de l'appareil de mesure de coordonnées, et que les données ainsi stockées sont utilisées sans aucune autre opération de calibrage pour les mesures suivantes avec le tomodensitomètre.

2. Procédé selon la revendication 1,
**caractérisé en ce**
**que** les réglages de grossissement et de plage de mesure calibrés au préalable sont automatiquement appelés par le programme de mesure de l'appareil de mesure de coordonnées et que les composants matériels correspondants sont automatiquement positionnés.

3. Procédé selon la revendication 1 ou 2,
**caractérisé en ce**
**que** la source de rayons X et le détecteur de rayons X sont déplacés en synchronisme afin de modifier uniquement le grossissement et/ou la plage de mesure.

4. Procédé selon la revendication 1 ou 2,

**caractérisé en ce**

**que** la source de rayons X et le détecteur de rayons X sont déplacés indépendamment l'un de l'autre afin de modifier le grossissement et/ou la plage de mesure.

5.  Procédé selon l'une des revendications 1 à 4,
    **caractérisé en ce**
    **que** tous les réglages requis pour la tomographie sont calibrés et enregistrés au préalable et que des opérations de calibrage ne sont plus nécessaires lors des opérations de tomographie respectives.

6.  Procédé selon l'une des revendications 1 à 5,
    **caractérisé en ce**
    **que** l'ajustage du centre de rotation de l'objet placé sur un plateau rotatif est réalisé par une opération de calibrage et/ou par une correction correspondante du décalage du centre de rotation dans le logiciel.

7.  Procédé selon la revendication 6,
    **caractérisé en ce**
    **que** la détermination du grossissement pour la tomographie et/ou la détermination de la position du centre de rotation par rapport à la source de rayons X et au détecteur de rayons X est/sont effectuée(s) au moyen d'un étalon constitué d'au moins deux billes.

8.  Procédé selon la revendication 6,
    **caractérisé en ce**
    **que** la détermination du grossissement pour la tomographie et/ou la détermination de la position du centre de rotation par rapport à la source de rayons X et au détecteur de rayons X est/sont effectuée(s) au moyen d'un étalon constitué de quatre billes.

9.  Procédé selon la revendication 8,
    **caractérisé par** les étapes suivantes pour déterminer la position du centre de l'axe de rotation dans l'appareil de mesure de coordonnées :

    - un étalon à quatre billes constitué de quatre billes disposées dans les angles d'un rectangle, tel qu'un carré, dans lequel les distances entre les billes sont connues et/ou calibrées, est positionné sur l'axe de rotation,
    - l'étalon à quatre billes est pivoté de manière telle que le plan engendré est parallèle au détecteur,
    - mesure de la position des quatre billes dans le champ de mesure du détecteur,
    - calcul du grossissement moyen M1 à partir des quatre distances entre billes mesurées, des distances nominales entre billes et de la taille nominale des pixels du détecteur,
    - rotation de 180° de l'axe de rotation,
    - mesure des quatre positions de bille dans l'image,
    - calcul du grossissement moyen M2 à partir des quatre distances entre billes mesurées, des distances nominales entre billes et de la taille nominale des pixels du détecteur.

10. Procédé selon la revendication 9,
    **caractérisé en ce**
    **que** le calcul de la position Y du centre de rotation est effectué à l'aide des quatre positions de bille avant et après la rotation selon l'équation suivante : Pdyn = (Pkyn1 * M2 + Pkyn2 + M1) / (M1 * M2), où Pdyn : position Y de l'axe de rotation sur le détecteur pour la bille n, Pkyn1 : position Y de la bille n sous l'angle de rotation 0°, Pkyn2 : position Y de la bille n sous l'angle de rotation 180°, M1 : grossissement moyen sous l'angle de rotation 0°, M2 : grossissement moyen sous l'angle de rotation 180°.

11. Procédé selon l'une des revendications 1 à 10,
    **caractérisé en ce**
    **que** dans l'appareil de mesure de coordonnées sont pris en compte des points de mesure mesurés sur l'objet à mesurer au moyen d'un système de détection tactile et/ou optique afin de corriger les points de mesure déterminés avec le tomodensitomètre.

12. Procédé selon la revendication 11,
    **caractérisé en ce**
    **qu'**est effectuée une correction du nuage de points de mesure de l'objet à mesurer mesuré avec la tomographie, ou des éléments de surface triangulés calculés à partir de celle-ci, par des points de mesure acquis par mesure tactile et/ou optique.

**13.** Procédé selon la revendication 12,
**caractérisé en ce**
**qu'**une interpolation est effectuée entre les points correctifs mesurés par mesure tactile et/ou optique.

**14.** Procédé selon la revendication 12,
**caractérisé en ce**
**qu'**une interpolation est effectuée entre les points correctifs acquis avec le système de détection tactile et/ou optique en tenant compte de l'évolution de la fonction du nuage de points mesuré par la tomographie et/ou en tenant compte d'un modèle nominal CAO.

**15.** Procédé selon l'une des revendications 1 à 14,
**caractérisé en ce**
**qu'**est tomographiée une partie calibrée d'un type d'objet à mesurer et qu'à partir de l'écart de mesure lors de la mesure est calculé un réseau correctif pour la correction des valeurs mesurées par tomographie, et que lors de la mesure de pièces de série, les mesures par tomographie sont accordées avec les valeurs correctives déterminées auparavant.

**16.** Procédé selon l'une des revendications 11 à 14,
**caractérisé en ce**
**que** tout d'abord une pièce modèle d'un type d'objet à mesurer est échantillonnée par mesure tomographique et tactile et/ou optique, qu'à partir de la différence des deux mesures est calculé un réseau correctif pour la correction des valeurs mesurées par tomographie et que lors de la mesure de pièces de série, les mesures par tomographie sont corrigées avec les valeurs correctives déterminées une seule fois.

**17.** Procédé selon la revendication 15 ou 16,
**caractérisé en ce**
**que** lors de la mesure de pièces de série sont en outre pris en compte des points correctifs individuels mesurés par mesure optique et/ou tactile.

**18.** Procédé selon la revendication 16 ou 17,
**caractérisé en ce**
**que** les points de mesure tactile et/ou optique pour la correction sont définis graphiquement par un opérateur sur le nuage de points déterminé par tomographie et sont ensuite automatiquement mesurés par l'appareil de mesure de coordonnées.

**19.** Procédé selon la revendication 16 ou 17,
**caractérisé en ce**
**que** les points de mesure tactile et/ou optique pour la correction sont définis graphiquement par un opérateur sur le modèle CAO de la pièce à mesurer et sont ensuite automatiquement mesurés par l'appareil de mesure de coordonnées.

**20.** Procédé selon la revendication 19,
**caractérisé en ce**
**que** les points de mesure tactile et/ou optique pour la correction sont répartis quasi uniformément ou uniformément sur la surface du modèle CAO par un algorithme automatique et automatiquement mesurés par l'appareil de mesure de coordonnées.

**21.** Procédé selon la revendication 19 ou 20,
**caractérisé en ce**
**que** les points de mesure tactile et/ou optique pour la correction sont définis à l'avance par un opérateur sur le modèle CAO et, après le chargement du modèle CAO, sont automatiquement mesurés par l'appareil de mesure de coordonnées.

**22.** Procédé selon l'une des revendications 6 à 21,
**caractérisé en ce**

**que** par principe lors de la tomographie, un corps étalon, en particulier un arrangement de billes, est également tomographié et que par ce moyen est déterminée la position relative de l'axe de rotation par rapport à l'appareil de mesure de coordonnées et/ou à la source de rayons X et/ou au capteur de rayons X, et que le grossissement effectif est ensuite mathématiquement corrigé.

**23.** Procédé selon la revendication 22,
**caractérisé en ce**
**que** le corps étalon, en particulier les billes de calibrage, est logé dans un support ayant de plus faibles propriétés d'absorption de rayons X que le corps étalon, et que l'objet à mesurer est positionné sur le plateau rotatif en tenant compte du corps étalon.

**24.** Procédé selon la revendication 22 ou 23,
**caractérisé en ce**
**que** la position du corps étalon sur l'axe de rotation est déterminée avec des capteurs optiques et/ou tactiles, et est prise en compte pour la correction de la position de l'axe de rotation.

**25.** Procédé selon l'une des revendications 22 à 24,
**caractérisé en ce**
**que** la position spatiale de l'axe de rotation par rapport à la source de rayons X et au détecteur de rayons X est déterminée métrologiquement avec le tomodensitomètre et/ou avec le système de détection tactile et/ou avec le système de détection optique, et que cet écart de position est mathématiquement corrigé lors de la tomographie de l'objet à mesurer.

**26.** Procédé selon l'une des revendications 6 à 25,
**caractérisé en ce**
**que** la position de l'axe de rotation s'écartant de la position nominale est corrigée par rotation et/ou translation et/ou distorsion des images 2D individuelles.

**27.** Procédé selon au moins une des revendications 6 à 26,
**caractérisé en ce**
**que** la position de l'axe de rotation s'écartant de la position nominale est prise en compte dans un algorithme de reconstruction.

**28.** Procédé selon l'une des revendications 6 à 27,
**caractérisé en ce**
**que** l'objet à mesurer est déterminé dans sa position sur le plateau rotatif de l'appareil de mesure et ainsi dans le système de coordonnées de l'appareil par la mise en oeuvre de capteurs tactiles et/ou optiques et/ou par tomographie, et est ensuite mesuré avec des méthodes de traitement d'images en mode de radioexposition 2D sur une position calibrée du capteur de rayons X.

**29.** Procédé selon l'une des revendications 1 à 28,
**caractérisé en ce**
**que** le tomodensitomètre est automatiquement piloté par le logiciel de l'appareil, sachant que le capteur de rayons X est positionné dans le cône de rayonnement de la source de rayons X pendant la tomographie et, en dehors de ces moments, est placé en position repos hors du cône de rayonnement.

**30.** Procédé selon l'une des revendications 1 à 29,
**caractérisé en ce**
**qu'**une technique sensorielle de traitement d'images en tant que deuxième système de détection et que le tomodensitomètre de l'appareil de mesure de coordonnées sont dotés sous forme d'un seul appareil de mesure de coordonnées multisensoriel du même matériel de traitement d'images et du même logiciel de traitement d'images et/ou de mêmes parties de celui-ci.

**31.** Procédé selon la revendication 30,
**caractérisé en ce**
**que** les méthodes de traitement d'images connues de la technique sensorielle de traitement d'images sont également applicables pour le tomodensitomètre.

Fig. 1

EP 1 749 190 B1

Fig. 2

Fig. 3

Fig. 5

Fig. 4

Fig. 6

Fig. 7

56  58  50
64  66  54
52
60
62

Fig. 8

136
300  302  304
134
158

Fig. 9

504 —⌇                                            500

                                                 502

Fig. 10

406

                                            402

a)

                                            400

406  404

406

                                            402

b)                                          408

406  404

406

                                            402

c)                                          410

404

Fig. 11

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 10331419 A
- DE 10044169 A
- DE 3806686 C
- EP 1380263 A
- US 20030043964 A

- DE 10001239 A
- DE 4445331 A
- EP 0504609 A
- US 5038378 A

**In der Beschreibung aufgeführte Nicht-Patentliteratur**

- *DE.Z.: Die Bibliothek der Technik,* vol. 248